# EUROPEAN PATENT SPECIFICATION

(11) **EP 1 827 284 B1**
(45) Date of publication and mention of the grant of the patent: **07.08.2013**
(21) Application number: 04806885.2
(22) Date of filing: 23.12.2004
(51) Int. Cl.: A61B 19/04, A61B 10/00, G01N 33/52, A61P 15/02

(54) **DEVICE AND METHOD FOR IDENTIFYING VAGINAL AFFECTIONS**
VORRICHTUNG UND VERFAHREN ZUR IDENTIFIZIERUNG VON VAGINALEN ERKRANKUNGEN
DISPOSITIF ET METHODE D'IDENTIFICATION DES AFFECTIONS VAGINALES

(43) Date of publication of application: 05.09.2007
(73) Proprietor: ACTIAL Farmaceutica Lda., Funchal (Madeira) 9000-082 (PT)
(72) Inventor: DE SIMONE, Claudio, I-00040 Ardea RM (IT)
(74) Representative: Cavattoni, Fabio
(86) International application number: PCT/IT2004/000720
(87) International publication number: WO 2006/080035

(56) References cited:
- EP-A- 0 956 858
- EP-A- 1 348 439
- WO-A-00/78322
- WO-A-84/04675
- WO-A-03/056954
- WO-A-2004/035072
- US-A- 6 123 676

## Description

The present invention relates to a device and a method using said device for the detection of vaginal affections, such as infections, in particular bacterial vaginosis, or vaginitis, in women by using an identifiable indicator. In particular, the present invention relates to a pH test glove bearing a reporter substance indicating the possible presence of vaginal affection.

### Background of the invention

The present invention relates to improving the health of women having vaginal affections and to procedures for determining the presence of a vaginal affection and, if present, topical use of a gynecological composition, preferably comprising *Lactobacillus* strains and/or *Bifidobacteria.* In a preferred aspect, the invention relates to a method for determining the presence of a vaginal affection in a subject using an identifiable indicator and, if the indication is positive, the use of a gynecological composition comprising one or more *Lactobacillus* strains and/or *Bifidobacteria* administered locally in an amount effective to treat the vaginal affection. Also described are packages or kits including a probe or tool such as a glove bearing a reporter substance indicating the presence of an affection packaged together with a gynecological composition for treating the vaginal affections, if it is found to be present.

The expression vaginal affections as used herein includes vaginal infections, such as bacterial vaginosis, fungal and viral infection, and vaginitis. Presence of a vaginal affection may be indicated by change in the pH of vaginal fluid, vaginal discharge, possible presence of indicator cells or strong or bad odor, vaginal discomfort, such as itching and burning. The gynecological compositions of the invention are also beneficial in the prevention or treatment of dyspareunia of which a vaginal infection is one of the causes.

Vaginitis is an inflammation of the lining of the vagina, which can be the result of a physical, chemical or biological agent. The present invention applies to every form of vaginitis.

Bacterial vaginosis (BV) is the most common vaginal disorder among reproductive age women, with prevalence from 10% to 40% in the entire female population, and from 5% to 50% among pregnant women.

BV is not caused by one specific pathogenic microorganism, but rather, by an imbalance of vaginal microbial flora.

Lactobacilli, that represent the prevalent microorganisms of the healthy human vagina, are reduced or absent in BV, particularly the H₂O₂-produci strains, and are replaced by *Gardnerella vaginalis* and other anaerobic microorganisms, such as *Bacteroides* spp., *Mobiluncus* spp., *Prevotella* spp., *Peptostreptococcus* spp., *Ureaplasma urealyticum,* and *Mycoplasma hominis,* most of which are normally found in the healthy vagina in small amounts.

Women with vaginal affection, in particular BV typically complain of vaginal discomfort and clinical symptoms, such as homogeneous malodorous vaginal discharge, more noticeable after unprotected intercourse, although some women are asymptomatic.

Often previously ignored or underestimated, the importance of vaginal flora in maintaining other aspects of health is now becoming increasingly understood.

Significant alterations in the vaginal microflora, such as those occurring in BV, have been associated with ascending infection, including pelvic inflammatory disease and obstetric complications such as premature rupture of membranes, amnionitis, post-partum endometritis, late miscarriage, and elevated risk of preterm birth.

Increasing data also suggest that abnormal vaginal flora may be a risk factor for recurrent urinary tract infection and the complications arising from BV include increased risk of sexually transmitted diseases, including human immunodeficiency virus.

Lactobacilli, particularly the H₂O₂ producing ones, play a pivotal role in controlling the microenvironment of the vagina and inhibiting the overgrowth of potentially pathogenic organisms. Possible mechanisms of this protection include inactivation of pathogens by different metabolic lactobacilli by-products (lactic acid, H₂O₂, bacteriocins), competition for epithelial cell attachment sites and stimulation of the local immune system. H₂O₂-produci strains of lactobacilli have been found in 70-96% of healthy women with normal flora, yet only 5% of women with BV.

BV infection is characterized by the presence of three of five criteria: release of an amine fishy odour, release of amine odour after addition of 10% potassium hydroxide solution, vaginal pH higher than 4.5, while the normal pH is between 3.8 and 4.5, presence of clue cells in the vaginal fluid, and milky homogenous vaginal discharge.

In most cases, the vaginal pH provides an early indication of any abnormality in the vaginal environment that often encourage the development of infections.

The increasing colonisation of the vagina by infectious bacteria often goes unnoticed by the mother-to-be. If an infection of this kind is detected early, prompt medical treatment can be given and the risk of premature birth reduced.

That is why it is so important to start measuring the pH of the vagina regularly from as early as possible in the pregnancy and to ascertain it is comprised between 3.8 and 4.5.

Different products to the purpose are available on the market.

Such products including disposable gynecological examination gloves, bearing thereon a pH-sensitive test indicator, such as CarePlan^{®} VpH gynecological examination gloves marketed by Selfcare Inc, are commercially available. Alternatively FemExam^{®}, a combined pH and amine test card is available from CooperSurgical, Inc.

The CarePlan^{®} VpH test is designed for measuring the acidity of the vaginal environment. This glove is coated with a pH indicator on the index finger and there is a colour scale on the back of the pack to read the test result. The problem with this product is that the index finger, coated with a pH indicator, is inserted in the vagina and in this way chemical substances could enter the vagina and cause inflammation and/or toxic damages. Moreover, excess mucus and/or discharges often present in the vagina remain on the glove finger and this excess could change the pH tester results.

The CarePlan^{®} VpH test is made of polyethylene, a low quality material, which is not adherent to the different size of different user's hand, therefore unsuitable for an accurate sampling in the vagina.

The FemExam^{®} is a credit-card size device having colorimetric test circular areas to distinguish pH 4.7 or greater as well as for the presence of volatile amines, both indicators of a bacterial vaginal infection.

In the use of this device, a cotton-tipped swab or similar arrangement is used to collect and apply a sample of vaginal fluid to the test areas. If present, elevated vaginal pH is noted and in a separate test area the presence of volatile amines is also reported.

The use of different components in the device, the swab and the card with test areas, can make the use of the device difficult. In addition, the swab could be of damage to the external orifice of the urethra or unwillingly left in the vagina.

Moreover the FemExam^{®} only distinguishes pH 4.7 or greater, whereas pH 4.5 is already a sign of the presence of BV, therefore a sign of infection can be lost by the subject making self-examination.

It is important to know that pH 4.5 is a landmark to distinguish a physiological condition from a pathological one.

Therefore, there is still the need of a device for self-examination for detecting a vaginal affection, which is easy to use, safe, accurate and capable of detecting early signs of the presence of an affection.

### Summary of the invention

The present invention provides an easy, risk-free, painless way to measure the vaginal pH, one of the most important signs to check for any abnormality in the vagina and for the presence and development of vaginal affection, such as vaginal infection and vaginitis, in particular bacterial vaginosis infections.

The present invention provides a glove bearing a reporter substance indicating the presence of a vaginal affection, said glove being characterized in that first means for detecting pH and second means for collecting a vaginal sample are provided thereof in separate positions.

By the construction of the glove according to the present invention, the self-examination can be done in an easy, relaxed way and, most important, no chemicals, such as those present on the pH detecting means according to the construction of gloves of the prior art, are introduced in the vagina. Moreover, the pH detecting means is not altered by the possible presence of excess mucus and/or discharges, which are often present in the vagina. There is no risk to harm vagina and no foreign material can be left into it.

### Detailed disclosure of the invention

According to the present invention, the glove is provided with first means for detecting pH and second means for collecting a vaginal sample, said means are arranged in a separate way on the glove in such a way the collecting means can be used without interfering with the means for detecting pH and said collecting means, having the vaginal sample thereon, can be subsequently contacted with said means for detecting pH.

According to the present invention, the term "provided" is used with the meaning that the first and second means are placed on the glove and "placed" means either "attached on" or "inserted in" or "coated on" or "embedded into".

In one embodiment of the present invention, said second means are placed or coated on one finger of the glove, preferably index or medium finger and said first means are placed on any position of the glove which can be contacted by the finger bearing said second means. For example, said first means can be placed on the thumb finger or on the surface of the glove in such a way the finger bearing said second means can be put into contact with said first means, for example on the palm or any other opposable finger.

Any other arrangement can be put into practice so as to allow said first and second means to be put into contact after vaginal sampling.

A preferred embodiment provides said first means placed on the thumb finger and said second means placed on the index or medium finger.

Any of the normally available pH-detecting means placed on the thumb finger can be used in the glove of the present invention, such as a normal litmus paper.

pH detection means can cover the full pH range. Conveniently, pH detection means are set to cover pH range from 3.8 to 4.5.
More conveniently, said pH detection means are set to detect values above pH 4.5.

A preferred embodiment of the present invention provides a mixture of pH indicator consisting of Methylorange and Bromocresol Green in the 1:5 (w/w) ratio. The color change range is between pH 3.8 and 4.5.

Any piece of information about pH reading can accompany the glove of the present invention, such as a colour scale. The piece of information can also contain warnings about pH range or value above which a medical advice is suggested. A pH value of 4.5 or higher is a warning for the affection, in particular BV.

The means for collecting vaginal sample, on an opposable finger, are made of a material suitable for collecting vaginal fluid and/or mucus. In a preferred embodiment of the present invention the material is an absorbent, non-woven, mono layer gauze.

In one of the possible embodiments of the present invention, the means for collecting vaginal sample are placed on the index finger.

In another possible embodiment of the present invention, the glove can be shaped in any different way than the five-finger glove. It is important that the glove bears pH detecting means and vaginal sample collecting means separately, i.e. in a way such that the collecting means and the detecting means do not come into contact during the sampling operation, but can be put into contact subsequently. For example a two-finger shaped glove, a thumb and a single part for all the remaining fingers, is a convenient embodiment.

Since the means for collecting vaginal sample and the means for detecting pH are separated, the former can be drained from excess of sample, thus providing more accurate pH detection.

The glove is made of any compatible material. A suitable material is low-density polyethylene (LDPE), but vinyl or latex are preferred materials. Conveniently, there is no need to provide sterile gloves and preferably, the glove is intended for single use.

In a possible embodiment of the invention, the glove is packed and each pack contains two hygienically packed test gloves, wherein the test paper has a range of pH detection from 3.8 to 4.5.

The glove according to the present invention can be equipped also with other devices suitable for performing other useful tests for determining the presence of a vaginal affection by using the same sample. For example, the glove can be equipped, either on the same glove or with a separate device, with a test for volatile amines, such as the one provided by FemExam^{®}. In alternative or in addition, other test devices can be provided, for example for determining the amount of lactic acid and/or to test the activity of enzymes (sialidase, prolidase, ect..) of interest as markers of a pathological state, and/or other biological indicators (e.g. cytokines, calprotectin) or markers of infection (viral, bacterial, fungine). Said tests are well-known to those skilled in the art and are normally available in clinical practice. As exemplary test, the one using tetrazolium salts as a redox active substance, is mentioned.

According to the present invention, the glove is suitable for the detection of vaginal affections. In a preferred embodiment of the invention, the detected vaginal affection is a vaginal infection, such as for example bacterial vaginosis, aerobic vaginitis, fungal infection or viral infection, or is vaginitis.

Another object of the present invention is a method for detecting a vaginal affection, said method comprising the steps of:
(a) securing a vaginal sample with the collecting means of the glove above described,
(b) contacting said collecting means with the pH detecting means,
(c) reading the exposed indicator signal substance and determining the predicted presence of a vaginal affection.

According to this method, collecting means, for example placed on index finger, must be inserted in the vagina and applied promptly on detecting means, placed on the thumb finger. The pH indicator changes its colour depending on the vaginal pH and the result should be immediately compared to a test paper, which can be contained in each pack. The absorbent gauze on the index finger is useful to absorb the excess mucus to be applied on the test strip. This way, no chemicals can go into the vagina and there are no risks of allergies or inflammation.

A normal pH does not mean total protection, but the method according to the present invention is particularly advisable if the woman has experienced vaginal infections and allows her to check if the acidity of her vagina is normal in between check-ups with her doctor.

The device according to the present invention is useful, convenient and more efficient with respect to other available products, such as the above mentioned CarePlan^{®} VpH and FemExam^{®}, in particular because the pH test paper come into close contact with the vagina, hence no chemicals are introduced into the vagina and there is no risk of allergies or inflammation. The result is immediately read thanks to the colour scale into the pack.

Once an abnormality or change in condition is observed and it is apparent therapy is advisable, treatment is instituted.

A further disclosure is a method of treating a vaginal affection comprising the steps of:
a) determining the presence of a vaginal affection by means of the glove above disclosed, and, if the affection is present,
b) topically applying to the affected area a gynecological composition.

Although any gynecological composition known in the art and suitable for the treatment of a vaginal affection can be used preferred active ingredients to combat the affection may be selected from a wide range of lactic acid bacteria and/or Bifidobacteria such as *Lactobacillus acidophilus, Lactobacillus buchneri, Lactobacillus casei, Lactobacillus catenaforme, Lactobacillus cellobiosus, Lactobacillus crispatus, Lactobacillus curvatus, Lactobacillus delbrueckii, Lactobacillus jensenii, Lactobacillus leichmannii, Lactobacillus minutus, Lactobacillus paracasei, Lactobacillus plantaruna, Lactobacillus rogosae, Lactobacillus salivarius, Lactobacillus brevis, Lactobacillus gasseri, Lactobacillus fermentum, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infantis, Bifidobacterium longum, Bifidobacterium plantarum., Bifidobacterium pseudo-catenulatum, Bifidobacterium pseudolongum, Streptococcus lactis, Streptococcus raffinolactis, Acidaminococcus fermenta, Cytophaga fermentans, Rhodoferax fermentans, Cellulomonas fermentans, Zymomonas mobilis,* and *Streptococcus thermophilus.*

Preferred, are those chosen from *Lactobacillus brevis* and *Lactobacillus salivarius* subsp. *salicinius* species optionally used in combination with one or more species of lactobacilli selected from *Lactobacillus salivarius* subsp. *salivarius, Lactobacillus jensenii, Lactobacillus catenaforme, Lactobacillus minutus, Lactobacillus gasseri* and *Lactobacillus plantarum* Mostly preferred is the combination consisting essentially of *Lactobacillus brevis, Lactobacillus salivarius* subsp. *salicinius* and *Lactobacillus plantarum*

Another preferred combination is the one consisting essentially of *Lactobacillus brevis, Lactobacillus casei, Lactobacillus salivarius subs. salicinius* and *Lactobacillus gasseri* species

Preferably, the association of bacteria used in the pharmaceutical composition comprises or consists essentially of *Lactobacillus brevis, Lactobacillus salivarius* subs. *saliciitius* and *Lactobacillus gasseri.* Particular examples of lactobacilli to be used are *Lactobacillus brevis Lactobacillus salivarius* subsp. *salicinius* and *Lactobacillus gasseri.*

Preferably, in the association of bacteria employed the bacteria concentration is 10⁷ to 10¹³ CFU/g, more preferably 10⁸ to 10¹² CFU/g, most preferably more than 10⁹ to 10¹² CFU/g. Preferably, in the association of bacteria each species is present at a concentration of 10⁸ to 10¹² CFU/g. The bacterial cultures preferably are in a lyophilized form.

The lactobacilli-containing compositions may also be used to treat every form of vaginitis, in particular, but not limited to, allergic vaginitis, aerobic vaginitis or other fungal vaginitis.

The package comprising the glove as above disclosed and the gynecological composition is used to prevent recurrence of bacterial vaginitis or vaginosis in women who were already affected by said disease and were treated with conventional therapy. The subject will check regularly vaginal pH and, in case a bacterial affection is detected, according to the instructions provided in the package or according the subject's own experience, an adequate treatment will be instituted. Any conventional treatment can be used. It is well known to use combined pharmacological treatment with antibacterial and antifungal drugs. Sterilization of vaginal environment or any other alteration of natural vaginal flora may be a side effect of this kind of treatment. In particular lactic acid bacteria naturally present in the vagina can succumb to the presence of antibacterial drugs. Moreover, if the lactobacilli are replaced by bacteria other than the anaerobes which are typical for BV, a distinct type of vaginitis may be seen. This condition is known as non-specific vaginitis, vaginitis of unknown origin, desquamative vaginitis, or, recently, aerobic vaginitis (AV). In cases of AV, aerobes such as *Escherichia coli* and Group B *streptococci (GBS)* are often isolated, and severe vaginal leukocytosis is accompanied by a foul smell and sticky discharge.

Adequate treatment methods are available for cases of classical anaerobic bacterial vaginosis, for both oral and intravaginal administration. Initial cure rates of 85 to 95 percent are achieved, but the long-term cure rate is low, with up to 80% recurrences within one year. For vaginitis with disrupted vaginal flora (AV), there is no uniform, efficient treatment method available, and the disease is usually enduring for many months to years. Oral penicillins and macrolides cause short-term relief, only to recur after antibiotic treatment is discontinued.

Both types of vaginitis involve a severe disruption of the Lactobacillus flora. Therefore, the re-introduction of vaginal *Lactobacillus acidophilus* may be a reasonable alternative to prevent recurrence of these conditions. Lactobacilli are believed to be the main factor in vaginal resistance against infection by other bacteria, commensals and pathogens alike. It has been proven that the loss of the Lactobacillus flora is linked to the symptoms of vaginitis, to an increased transmission risk of sexually transmitted diseases, especially HIV, and to pregnancy complications such as pre-term birth. Lactobacillary grades can be used as a pre-screening tool during pregnancy, in order to enable further investigation of cases with a high probability of infection and pre-term delivery.

Preliminary evidence suggests that a disrupted vaginal microflora can be restored to its physiological equilibrium by the application of vaginal tablets containing probiotics. However, it is far from clear which type of probiotic product is to be used, and the ideal way of applying the products in order to prevent recurrences are also largely unknown.

Once a vaginal affection is detected, adequate treatment is instituted. The treatment comprises the combined administration of an antibacterial agent and an antifungal agent, for example metronidazole and fuconazole, respectively. A suitable treatment is for example 2 g of metronidazole orally and 200 mg of fluconazole orally. Other kind of administrations can be used, for example topical application, such as creams, ointments, pessaries, tablets, ovulae. After treatment, as determined according to medical advice or subject's own experience, a suitable re-colonization treatment is instituted with a composition containing lactic acid bacteria as above disclosed. During the treatment, vaginal pH will be self-checked on a regular basis until restoration of a normal pH, say from about 3.8 to about 4.5 by means of the glove of the present invention. An example of suitable treatment is one vaginal tablet every second day for 8 days (4 tablets in total) containing viable lactobacilli according to the present invention. Insertion should be performed after menses or, in women with amenorrhea, monthly, in the evening before retiring. If the onset of menstruation recurs during the treatment period, treatment should be interrupted and resumed after menstruation has ceased. The treatment can be stopped once vaginal pH is set in the physiological interval (from about 3.8 to about 4.5).

Therefore, a method for the treatment and re-colonization of vaginal environment is disclosed, said method comprising:
a) determining the presence of a vaginal affection by means of the glove above disclosed, and, if the affection is present,
b) topically applying to the affected area a gynecological composition in order to treat the vaginal affection,
c) checking the disappearance of the vaginal affection, and
d) topically applying a gynecological composition comprising lactic acid bacteria, checking vaginal pH by means of the glove above disclosed.

The gynecological composition comprising lactic acid bacteria and/or Bifidobacteria can be any composition disclosed. One of the preferred compositions comprises the combination of the following strains: *Lactobacillus brevis, Lactobacillus salivarius* subsp. *salicinius* and *Lactobacillus plantarum.*

According to this embodiment, the present invention also provides a package comprising:
a) at least one glove;
b) a gynecological composition for the treatment of a vaginal affection;
c) a gynecological composition comprising lactic acid bacteria and/or Bifidobacteria; and
d) instructions for using the glove a) the composition b) and the composition c).

A convenient embodiment of the present invention provides a package containing:
a) a composition comprising a combination of an antibiotic agent, for example metronidazole, with an antifungal agent, for example fuconazole, in a dosage form suitable for treating a vaginal affection, such as bacterial vaginosis or aerobic vaginitis;
b) a gynecological composition for topical application comprising lactic acid bacteria and/or Bifidobacteria in a dosage form suitable for re-colonizing vaginal environment;
c) a sufficient number of gloves according to the present invention to detect a vaginal affection, to check effectiveness of treatment instituted with the composition under item a) and to check effectiveness of treatment instituted with the composition under item b); and
d) instructions for using the package.

This form of package is suitable for women experiencing vaginal affection and wishing to adopt a self-treatment. This package can be very convenient for travelling women or for women who cannot see a doctor very often or who are experienced with relapsing vaginal affections and have sufficient experience in self diagnosis and self-treatment. Another therapeutic product useful for treating vaginal conditions, particularly vaginal infections, is tea tree oil, a natural product extracted from Melaleuca Alternifolia leaves, an oil extracted by distillation containing tupinene-4-ol. Tea tree oil may be taken orally in a solubilized aqueous solution or applied topically or in a vaginal tablet when included in the appropriate formulating agent(s).

Other products of use for the vaginal disorders are soy and its derivatives. Soy contains phytoestrogens, natural occurring polyphenols which can be of help in the treatment of postmenopausal hormonal disorders, including osteoporosis.

For practical use the compositions used including gynecological, cosmetic and pharmaceutical compositions, are prepared in any convenient form for topical application such as in a liquid form, in the form of creams or ointments, or in a solid form, i.e. as pessaries or vaginal tablets, packets and the like. The compositions made in the form of vaginal tablets can be of at least one layer or at least two layers having differentiated release times.

The compositions can be prepared in the form of tablets made up of two or more layers. Tea tree oil and/or soy may also be included in one or both layers of the tablet. Such two layers, both containing a species of lactobacilli, bound with usual excipients and additives, can be arranged in such a manner that bacteria in the outer layer are released in a lapse of time of 10-25 minutes, about 15-20 minutes for example, whereas bacteria of the inner layer are released subsequently in a lapse of time of 25-50 minutes, about 30-40 minutes for example.

The compositions may also contain a buffering agent capable of maintaining an intravaginal pH stabilized in a range between 3 and 5.5 for some hours after administration. The buffering agent is a buffer system consisting of a weak acid selected from any pharmaceutically-acceptable inorganic or organic weak acid, such as boric acid, lactic acid, ascorbic acid, citric acid or acetic acid for example, in combination with the respective sodium salt or another pharmaceutically acceptable salt of the conjugated base of the weak acid used. Preferably, the pH is buffered in a range of 4.2 to 4.5 and preferably the buffer agent used is a buffer system made up of lactic acid and sodium lactate or ascorbic acid and sodium ascorbate. These and related compositions are described in De Simone et al U.S. 6,277,370 the entire content of which is incorporated by reference.

The pharmaceutical compositions used for therapy herein are described in De Simone WO 00/78322 A2 and its counterpart U.S. application Serial No. 10/024,199 filed December 21, 2001. This embodiment employs a combination of lactic acid bacteria including (a) a first component consisting of at least one strain of an H₂O₂-producing lactic acid bacteria and (b) a second component consisting of a strain of arginine-utilizing bacteria.

The lactic acid bacteria in component (b) is the *Lactobacillus brevis* CD2 strain deposited under the Budapest Treaty under accession no. DSM 11988. The ratio of the amounts of bacteria in components (a):(b) will range from 100:1 to 1:100, preferably 1:5 to 5:1 and desirably in a substantially equal ratio of 1:1. Generally a unit dosage will contain 1 x 10² to 5 x 10¹¹ bacteria of component (a) and from 1 x 10² to 5 x 10¹¹ bacteria of component (b) with preferred amounts being 1 x 10⁹ bacteria for component (a) and 3 x 10⁹ bacteria of component (b).

A preferred combination of lactic acid bacteria contains (a) a first component consisting of at least one strain of H₂O₂-producing lactic acid bacteria, and (b) a second component consisting of at least one strain of arginine-utilizing lactic acid bacteria, where component (a) is selected from strains of the species *Lactobacillus crispatus, Lactobacillus sali-various,* preferably subsp. *salicinius* and *Lactobacillus casei,* and component (b) is selected from strains of the species *Lactobacillus brevis, Lactobacillus gasseri* and *Lactobacillus fermentum.*

This particular combination of H₂O₂-produci lactic acid bacteria and arginine-utilizing bacteria can optionally comprise one or more of the following strains *Lactobacillus salivarius subs. salivarius, Lactobacillus jensenii, Lactobacillus catenafornie, Lactobacillus minutus,* and *Lactobacillus plantarum.*

Particularly preferred compositions are when component (a) is *Lactobacillus crispatus* and component (b) is *Lactobacillus brevis* or when component (a) is *Lactobacillus salivarius* and component (b) is *Lactobacillus brevis.*

Additionally vitamins, quaternary ammonium bases, mineral salts, tea tree oil, soy and its derivatives, antioxidant agents, antiinflammatory, antihistamine and NO-releasing substances (such as sildenafil) may also be present.

A procedure for identifying and treating a vaginal affection, comprises the steps of:
(a) securing a sample of vaginal fluid,
(b) exposing the collected fluid to an indicator signal substance,
(c) reading the exposed indicator signal substance and determining the predicted presence of a vaginal affection in said sample, wherein steps (a)-(c) are made by means of the glove herein disclosed, and if an affection is present,
(d) applying to the area of affection a gynecological composition.

The preferred composition comprises lactic acid bacteria and/or Bifidobacteria as disclosed above.

As far as the industrial applicability is concerned, the above glove and gynecological composition can be provided in the form of a package comprising:
a) at least one glove;
b) a gynecological composition;
c) instructions for using the glove a) and the composition b).

It is intended that "a glove" of item a) in the package can be one single glove or a set of gloves. The number of gloves can be suitable determined for carrying out the method for detecting a vaginal affection, and, if present, instituting a treatment of the affection with the composition b), and using the remaining gloves periodically in order to check the effect of the treatment, until disappearance of the affection, detected with a normal pH, such as above 4.5.

Instructions as per item c) are not bound to any particular form. They essentially provide guidance to the user for interpreting the results given by the indicator substance after having contacted the vagina sample secured with the collecting means provided on the glove.

A first item of instruction is an interpretative reading of pH, conveniently by means of a colour scale.

A second item of instruction is a correlation between the pH reading and the health condition or possible presence of an affection.

For example, a pH between 3.8 and 4.5, with no signs or symptoms means a normal and healthy condition. A pH lower than 3.8, together with whitish discharge and/or symptoms, such as burning and itching means a possible *Candida* vaginal infection. A pH higher than 4.5, with no signs or symptoms means a vaginal microenvironment, which is potentially favourable to the development of vaginal infections. A pH higher than 4.5, together with possibly malodorous vaginal discharges means dismicrobism with reduced or missing H₂O₂-producing lactic acid bacteria (bacterial vaginosis). A pH higher than 4.5, together with possibly malodorous vaginal discharges and/or irritation, itching means a possible infection by *Trichomonas vaginalis.*

Once affection is identified, the treatment can be instituted by means of the gynecological composition, in particular the one containing lactic acid bacteria and/or Bifidobacteria, as above disclosed. In some cases, such as suspected *Candid albicans* or *Trichomonas vaginalis* infection, a medical advice is suggested.

Normally the user will see a doctor, who will decide the posology for using the composition. However, a woman who already experienced vaginal affection can self-administer the gynecological composition provided in the package, according to her experience.

A possible treatment can be, for example one vaginal application, for example a tablet, per day, for 8 consecutive days, or, alternatively, one vaginal application every second day for 16 days.

Typically, a package can contain 8 vaginal tablets, two gloves and instructions for use.

For the purposes of industrial applicability, the presen invention also relates to the use of the glove as above disclosed and of the compositions comprising lactic acid bacteria and/or Bifidobacteria. for the detection of a vaginal affection A further object of the present invention is the use of the glove as above disclosed and of the compositions comprising lactic acid bacteria and/or Bifidobacteria and another composition used in the treatment of bacterial vaginisis or aerobic vaginitis, as above described for the detection of a vaginal affection, treatment thereof and re-colonization of vaginal environment.

While the invention has been described in connection with what is presently considered to be the most practical and preferred embodiment, it is to be understood that the invention is not to be limited to the disclosed embodiment, but on the contrary, is intended to cover various modifications and equivalent arrangements included within scope of the appended claims.

## Claims

1. A glove for detecting the presence of a vaginal affection, said glove being provided with first means for detecting pH and second means for collecting a vaginal sample, the glove being **characterized in that** said first means are provided with a reporter substance for measuring pH, said second means for collecting vaginal sample are placed on one finger of the glove, and said first and second means are placed in separate positions on the glove.

2. The glove according to claim 1, wherein said means for detecting pH are placed on thumb finger and said second means are placed on the index finger or medium finger.

3. The glove according to any one of claims 1-2, wherein said means for detecting pH are set to cover pH range from 3.8 to 4.5 or above 4.5.

4. The glove according to claim 3, wherein said means for detecting pH is a mixture of Methylorange and Bromocresol Green in a 1:5 (w/w) ratio.

5. The glove according to any one of claims 1-4, wherein a piece of information about pH reading is enclosed.

6. The glove according to claim 5, wherein said piece of information is a colour scale.

7. The glove according to claim 4, wherein said values equal to or above 4.5 is set as a warning.

8. The glove according to any one of claims 1-7, wherein said means for collecting vaginal sample are made of a material suitable for collecting vaginal fluid and/or mucus.

9. The glove according to claim 8, wherein said means material is an absorbent, non-woven, mono layer gauze.

10. The glove according to any one of claims 1-9, which is made of vinyl or latex.

11. The glove according to any one of claims 1-10, further comprising a separate device for the determination of a further test useful for determining the presence of a vaginal affection.

12. The glove according to claim 11, wherein said further test is selected from the group consisting of a test for volatile amines, test for determining the amount of lactic acid, test for determining the activity of enzymes of interest as markers of a pathological state and/or other biological indicators and test for determining the presence of markers of infection.

13. The glove according to any one of the preceding claims 1-12, which is useful for determining the presence of a vaginal affection selected from the group consisting of vaginal infection and vaginitis.

14. The glove according to claim 13, wherein said vaginal infection is selected from the group consisting of bacterial vaginosis, aerobic vaginitis, fungal infection and viral infection.

15. A package comprising: a) at least one glove of any one of claims 1-14 and instructions for its use.

16. The package according to claim 15, further comprising:
b) a gynecological composition; and optionally
c) instructions for using the glove a) and the composition b).

17. The package according to claim 16, wherein said composition (b) comprises lactic acid bacteria and/or Bifidobacteria selected from the group consisting of *Lactobacillus acidophilus, Lactobacillus buchneri, Lactobacillus casei, Lactobacillus catenaforme, Lactobacillus cellobiosus, Lactobacillus crispatus, Lactobacillus curvatus. Lactobacillus delbrueckii, Lactobacillus jensenii, Lactobacillus leichmannii, Lactobacillus minutus, Lactobacillus paracasei, Lactobacillus plantarum, Lactobacillus rogosae, Lactobacillus salivarius. Lactobacillus brevis, Lactobacillus gasseri, Lactobacillus fermentum, Bifidobacterium adolescentis, Bifidobacterium angulatum, Bifidobacterium bifidum, Bifidobacterium breve, Bifidobacterium catenulatum, Bifidobacterium dencium, Bifidobacterium eriksonii, Bifidobacterium infantis, Bifidobacterium longum, Bifidobacterium plantarum, Bifidobacterium pseudo-catereulatum, Bifidobacterium pseudolongum, Streptococcus lactis, Streptococcus raffinolactis, Acidaminococcus fermenta, Cytophaga fermentans, Rhodoferax fermentans, Cellulomonas fermentans, Zymomonas mobilis,* and *Streptococcus thermophilus.*

18. The package according to claim 17, wherein the lactic acid bacteria are selected from the group consisting of *Lactobacillus brevis* and *Lactobacillus salivarius,* subsp. *salicinius* species optionally in combination with one or more species of lactobacilli selected from the group consisting of *Lactobacillus salivarius ,* subsp. *salivarius, Lactobacillus jensenii, Lactobacillus catenaforme, Lactobacillus minutus. Lactobacihus gasseri* and *Lactobacillus plantarum.*

19. The package according to claim 18, wherein the lactic acid bacteria are a combination consisting of *Lactobacillus brevis, Lactobacillus salivarius* subsp. *salicinius* and *Lactobacillus plantarum.*

20. The package according to claim 17, wherein the lactic acid bacteria are a combination of *Lactobactllus brevis* and *Lactobacillus salivartus subs. salicinius species.*

21. The package according to claim 17, wherein said lactic acid bacteria is a combination of (a) a first component consisting of at least one strain of H₂O₂-producing lactic acid bacteria, and (b) a second component consisting of at least one strain of arginine-utilizing lactic acid bacteria

22. The package according to claim 21, wherein said lactic acid bacteria is (a) selected from the group consisting of *Lactobacillus crispatus, Lactobacillus salivarius* and *Lactobacillus casei,* and component (b) is selected from strains of the species *Lactobacillus brevis. Lactobacillus gasseri* and *Lactobacillus fermentum.*

23. The package according to claim 21, wherein the lactic acid bacteria are a combination of the *Lactobacillus brevis* and *Lactobacihus salivarius* subs. *salicinius* species.

24. The package according to any one of claims 17-23, wherein the lactic acid bacterium is *Lactobacillus brevis* CD2 DSM 11988.

25. The package according to any one of claims 21-24, wherein said composition further comprises one or more species of lactobacilli selected from the group consisting of *Lactobacillus salivarius subs. salivarius, Lactobacillus jensenii, Lactobacillus catenaforme. Lactobacillus minutus,* and *Lactobacillus plantarum.*

26. The package according to claim 17, wherein said lactobacilli consist of the *Lactobacillus brevis, Lactobacillus casei. Lactobacillus salivarius subs. salicinius* and *Lactobacillus gasseri species.*

27. The package according to any one of claims 17-26, wherein said lactobacilli are present at a concentration of 10⁷ to 10¹³ CFU/g.

28. The package according to any one of claims 17-27, wherein the composition further comprises at least a substance selected from the group consisting of vitamins, quaternary ammonium bases, mineral salts, tea tree oil, soy and its derivatives, antioxidant agents, antiinflammatory agents, antihistamine agents and NO-releasing substance.

29. The package according to claim 17, wherein the composition (b) comprises at least a substance selected from the group consisting of tea tree oil and/or soy and its derivatives.

30. The package according to claim 29, wherein the composition further comprises at least a substance selected from the group consisting of vitamins, quaternary ammonium bases, mineral salts and antioxidant agents.

31. The package according to any one of claims 17-30, wherein said composition is in a liquid form or a cream, ointment, pessary, vaginal tablet or packet.

32. The package according to claim 31, wherein said vaginal tablet comprises at least one layer of lactobacilli, in bound form, to control the release velocity of bacteria.

33. The package of claim 32, wherein said tablet comprises at least two layers, the outermost layer and the innermost layer containing said species of lactobacilli, in bound form, so that the release velocity of bacteria of the outermost layer is greater than the release velocity of bacteria of the innermost layer.

34. The package of claim 33, wherein in said tablet, bacteria in the outermost layer are released in a lapse of time of about 10-25 minutes, whereas bacteria of the inner layer are released subsequently in a lapse of time of about 25-50 minutes.

35. The package according to any one of claims 16-34, further comprising (d) a composition for the treatment of bacterial vaginosis or aerobic vaginitis and for the re-colonization of vaginal enviromnent.

36. The package according to claim 35, wherein said composition (d) comprises a combination of an antibacterial agent and an antifungal agent.

37. The package according to claim 36, wherein said antibacterial agent is metronidazole and said antifungal agent is fuconazole.

38. The package according to any of claims 35-37, further comprising instructions for the use of the glove (a), composition (b) and composition (d).

39. A method for determining the presence of a vaginal affection, comprising the steps of:
(i) contacting the collecting means of the glove of any one of claims 1-14 that has been used to secure the vaginal sample with the pH detecting means,
(ii) reading the exposed indicator signal substance and determining the predicted presence of said vaginal affection.

40. The method according to claim 39, wherein said affection is selected from the group consisting of vaginal infections, vaginitis and dyspareunia.

41. The method according to claim 40, wherein said infection is selected from the group consisting of bacterial vaginosis, aerobic vaginitis, fungal infection and viral infection.

42. Use of the glove of any one of claims 1-14 for the detection treatment of a vaginal affection.

43. Use of the package of any one of claims 15-38 for the detection of a vaginal affection,

44. The use according to claim 43, wherein said affection is selected from the group consisting of vaginal infections, vaginitis and dyspareunia.

45. The use according to claim 44, wherein said infection is selected from the group consisting of bacterial vaginosis, aerobic vaginitis, fungal infection and viral infection.

## Patentansprüche

1. Handschuh zur Erkennung der Präsenz einer Vaginalerkrankung, wobei der Handschuh mit einem ersten Mittel/Mitteln zur Erkennung des pHs und einem zweiten Mittel/Mitteln zum Einsammeln einer Vaginalprobe bereitgestellt wird, wobei der Handschuh dadurch charakterisiert ist, dass die ersten Mittel mit einer Reportersubstanz zur Messung des pHs bereitgestellt werden, dass die zweiten Mittel zum Einsammeln der Vaginalprobe an einem Finger des Handschuhs platziert sind und dass das erste und zweite Mittel/die ersten und zweiten Mittel an unterschiedlichen Stellen an dem Handschuh platziert sind.

2. Handschuh gemäß Anspruch 1, wobei das/die Mittel zur Erkennung des pHs am Daumenfinger platziert ist/sind und die zweiten Mittel am Zeigefinger oder Mittelfinger platziert sind.

3. Handschuh gemäß einem der Ansprüche 1-2, wobei die Mittel zur Erkennung des pHs eingestellt sind einen pH Bereich von 3,8 bis 4,5 oder mehr als 4,5 abzudecken.

4. Handschuh gemäß Anspruch 3, wobei das Mittel zur Erkennung des pHs eine Mischung aus Methylorange und Bromkresolgrün in einem Gewichtsverhältnis von 1:5 ist.

5. Handschuh gemäß einem der Ansprüche 1-4, wobei eine Information zur Ablesung des pHs beigefügt ist.

6. Handschuh gemäß Anspruch 5, wobei die Information eine Farbskala ist.

7. Handschuh gemäß Anspruch 4, wobei die Werte gleich oder über 4,5 als eine Warnung bestimmt sind.

8. Handschuh gemäß einem der Ansprüche 1-7, wobei die Mittel zum Einsammeln einer Vaginalprobe aus einem Material hergestellt sind, welches dazu geeignet ist Vaginalflüßigkeit und/oder Schleim einzusammeln.

9. Handschuh gemäß Anspruch 8, wobei das Mittelmaterial eine absorbierende, nicht-gewebte, einschichtige Gaze ist.

10. Handschuh gemäß einem der Ansprüche 1-9, welcher aus Vinyl oder Latex hergestellt ist.

11. Handschuh gemäß einem der Ansprüche 1-10, welcher weiterhin eine getrennte Vorrichtung zur Bestimmung eines weiteren Tests, der geeignet ist zur Bestimmung der Präsenz einer Vaginalerkrankung, umfasst.

12. Handschuh gemäß Anspruch 11, wobei der weitere Test ausgewählt ist aus der Gruppe bestehend aus einem Test für volatile Amine, einem Test zur Bestimmung der Menge an Milchsäure, einem Test zur Aktivitätsbestimmung von Enzymen, welche als Marker eines pathologischen Zustands von Interesse sind und/oder anderen biologischen Indikatoren, und einem Test zur Bestimmung der Präsenz von Infektionsmarkern.

13. Handschuh gemäß einem der vorangehenden Ansprüche 1-12, welcher zur Bestimmung der Präsenz einer Vaginalerkrankung ausgewählt aus der Gruppe bestehend aus Vaginalinfektion und Vaginitis geeignet ist.

14. Handschuh gemäß Anspruch 13, wobei die Vaginalinfektion ausgewählt ist aus der Gruppe bestehend aus bakterielle Vaginose, aerobe Vaginitis, Pilzinfektion und Virusinfektion.

15. Packung umfassend: a) mindestens einen Handschuh gemäß einem der Ansprüche 1-14 und eine Anleitung für dessen Gebrauch.

16. Packung gemäß Anspruch 15, weiterhin umfassend:
b) eine gynäkologische Zusammensetzung und optional
c) eine Gebrauchsanleitung für den Handschuh a) und die Zusammensetzung b).

17. Packung gemäß Anspruch 16, wobei die Zusammensetzung (b)Milchsäurebakterien und/oder Bifidobakterien ausgewählt aus der Gruppe bestehend aus Lactobacillus acidophilus, Lactobacillus buchneri, Lactobacillus casei, Lactobacillus catenaforme, Lactobacillus cellobiosus, Lactobacillus crispatus, Lactobacillus curvatus, Lactobacillus delbrueckii, Lactobacillus jensenii, Lactobacillus leichmannii, Lactobacillus minutus, Lactobacillus paracasei, Lactobacillus plantarum, Lactobacillus rogosae, Lactobacillus salivarius, Lactobacillus brevis, Lactobacillus gasseri, Lactobacillus fermentum, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infantis, Bifidobacterium longum, Bifidobacterium plantarum, Bifidobacterium pseudo-catenulatum, Bifidobacterium pseudolongum, Streptococcus lactis, Streptococcus raffinolactis, Acidaminococcus fermenta, Cytophaga fermentans, Rhodoferax fermentans, Cellulomonas fermentans, Zymomonas mobilis und Streptococcus thermophilus umfasst.

18. Packung gemäß Anspruch 17, wobei die Milchsäurebakterien ausgewählt sind aus der Gruppe bestehend aus den Spezies Lactobacillus brevis und Lactobacillus salivarius subsp. salicinius, optional in Kombination mit einer oder mehreren Spezies von Laktobazillen ausgewählt aus der Gruppe bestehend aus Lactobacillus salivarius subsp. salivarius, Lactobacillus jensenii, Lactobacillus catenaforme, Lactobacillus minutus, Lactobacillus gasseri und Lactobacillus plantarum.

19. Packung gemäß Anspruch 18, wobei die Milchsäurebakterien eine Kombination bestehend aus Lactobacillus brevis, Lactobacillus salivarius subsp. salicinius und Lactobacillus plantarum sind.

20. Packung gemäß Anspruch 17, wobei die Milchsäurebakterien eine Kombination der Spezies bestehend aus Lactobacillus brevis und Lactobacillus salivarius subsp. salicinius sind.

21. Packung gemäß Anspruch 17, wobei die Milchsäurebakterien eine Kombination aus (a) einer ersten Komponente bestehend aus mindestens einem H₂O₂-produzierendem Milchsäurebakterienstamm und (b) einer zweiten Komponente bestehend aus mindestens einem Argininverwertendem Milchsäurebakterienstamm sind.

22. Packung gemäß Anspruch 21, wobei die Milchsäurebakterien (a) ausgewählt sind aus der Gruppe bestehend aus Lactobacillus crispatus, Lactobacillus salivarius und Lactobacillus casei und Komponente (b) ausgewählt ist aus Stämmen der Spezies Lactobacillus brevis, Lactobacillus gasseri und Lactobacillus fermentum.

23. Packung gemäß Anspruch 21, wobei die Milchsäurebakterien eine Kombination der Spezies Lactobacillus brevis und Lactobacillus salivarius subsp. salicinius sind.

24. Packung gemäß einem der Ansprüche 17-23, wobei das Milchsäurebakterium Lactobacillus brevis CD2 DSM 11988 ist.

25. Packung gemäß einem der Ansprüche 21-24, wobei die Zusammensetzung weiterhin eine oder mehrere Spezies von Laktobazillen ausgewählt aus der Gruppe bestehend aus Lactobacillus salivarius subsp. salivarius, Lactobacillus jensenii, Lactobacillus catenaforme, Lactobacillus minutus und Lactobacillus plantarum umfasst.

26. Packung gemäß Anspruch 17, wobei die Laktobazillen aus den Spezies Lactobacillus brevis, Lactobacillus casei, Lactobacillus salivarius subsp. salicinius und Lactobacillus gasseri bestehen.

27. Packung gemäß einem der Ansprüche 17-26, wobei die Laktobazillen mit einer Konzentration von 10⁷ bis 10¹³ CFU/g vorhanden sind.

28. Packung gemäß einem der Ansprüche 17-27, wobei die Zusammensetzung weiterhin mindestens eine Substanz ausgewählt aus der Gruppe bestehend aus Vitaminen, quartären Ammoniumbasen, Mineralsalzen, Teebaumöl, Soja und dessen Derivaten, Antioxidationsmitteln, antiinflammatorischen Mitteln, antihistaminischen Mitteln und NO-freisetzenden Substanzen umfasst.

29. Packung gemäß Anspruch 17, wobei die Zusammensetzung (b) mindestens eine Substanz ausgewählt aus der Gruppe bestehend aus Teebaumöl und/oder Soja und dessen Derivaten umfasst.

30. Packung gemäß Anspruch 29, wobei die Zusammensetzung weiterhin mindestens eine Substanz ausgewählt aus der Gruppe bestehend aus Vitaminen, quartären Ammoniumbasen, Mineralsalzen und Antioxidationsmitteln umfasst.

31. Packung gemäß einem der Ansprüche 17-30, wobei die Zusammensetzung in einer flüssigen Form oder einer Creme, einer Salbe, einem Pessar, einer Vaginaltablette oder einem Paket ist.

32. Packung gemäß Anspruch 31, wobei die Vaginaltablette mindestens eine Schicht aus Laktobazillen in gebunder Form umfasst, um die Freisetzungsgeschwindigkeit der Bakterien zu kontrollieren.

33. Packung gemäß Anspruch 32, wobei die Tablette mindestens zwei Schichten umfasst, wobei die äußerste Schicht und die innerste Schicht die Spezies der Laktobazillen in gebundener Form enthalten, so dass die Freisetzungsgeschwindigkeit der Bakterien der äußersten Schicht größer ist als die Freisetzungsgeschwindigkeit der Bakterien der innersten Schicht.

34. Packung gemäß Anspruch 33, wobei in der Tablette die Bakterien in der äußersten Schicht in einem Zeitraum von ungefähr 10-25 Minuten freigesetzt werden, wohingegen die Bakterien der inneren Schicht danach in einem Zeitraum von ungefähr 25-50 Minuten freigesetzt werden.

35. Packung gemäß einem der Ansprüche 16-34, weiterhin umfassend (d) eine Zusammensetzung zur Behandlung bakterieller Vaginose oder aerober Vaginitis und zur Wiederbesiedelung der vaginalen Umgebung.

36. Packung gemäß Anspruch 35, wobei die Zusammensetzung (d) eine Kombination aus einem antibakteriellen Mittel und einem antimykotischen Mittel umfasst.

37. Packung gemäß Anspruch 36, wobei das antibakterielle Mittel Metronidazol und das antimykotische Mittel Fuconazol ist.

38. Packung gemäß einem der Ansprüche 35-37, weiterhin umfassend eine Gebrauchsanleitung für den Handschuh (a), die Zusammensetzung (b) und die Zusammensetzung (d).

39. Verfahren zur Bestimmung der Präsenz einer Vaginalerkrankung umfassend die Schritte:
(i) in Kontakt bringen des Einsammelmittels des Handschuhs gemäß einem der Ansprüche 1-14, welches dazu benutzt wurde eine Vaginalprobe zu sichern, mit dem pH Erkennungsmittel/Erkennungsmitteln,
(ii) Ablesen der exponierten Indikatorsignalsubstanz und Bestimmung der vorhergesagten Präsenz der Vaginalerkrankung.

40. Verfahren gemäß Anspruch 39, wobei die Erkrankung ausgewählt ist aus der Gruppe bestehend aus Vaginalinfektionen, Vaginitis und Dyspareunie.

41. Verfahren gemäß Anspruch 40, wobei die Infektion ausgewählt ist aus der Gruppe bestehend aus bakterieller Vaginose, aerober Vaginitis, Pilzinfektion und viraler Infektion.

42. Verwendung des Handschuhs gemäß einem der Ansprüche 1-14 zur Erkennung einer Vaginalerkrankung.

43. Verwendung der Packung gemäß einem der Ansprüche 15-38 zur Erkennung einer Vaginalerkrankung.

44. Verwendung gemäß Anspruch 43, wobei die Erkrankung ausgewählt ist aus der Gruppe bestehend aus Vaginalinfektionen, Vaginitis und Dyspareunie.

45. Verwendung gemäß Anspruch 44, wobei die Infektion ausgewählt ist aus der Gruppe bestehend aus bakterieller Vaginose, aerober Vaginitis, Pilzinfektion und viraler Infektion.

## Revendications

1. Un gant pour détecter la présence d'affection vaginale, ledit gant étant pourvu de premiers moyens pour détecter le pH et de seconds moyens pour prélever un échantillon vaginal, le gant étant **caractérisé en ce que** les premiers moyens sont pourvus d'une substance rapporteuse pour mesure le pH, lesdits seconds moyens pour prélever un échantillon vaginal sont placés sur un doigt du gant, et lesdits premiers et seconds moyens sont placés à des positions séparées sur le gant.

2. Le gant selon la revendication 1, dans lequel lesdits moyens pour détecter le pH sont placés sur le pouce et lesdits seconds moyens sont placés sur l'index ou sur le medium.

3. Le gant selon l'une quelconque des revendications 1 ou 2, dans lequel lesdits moyens pour détecter le pH sont fixés pour couvrir des gammes de pH de 3,8 à 4,5 ou au-dessus de 4,5.

4. Le gant selon la revendication 3, dans lequel lesdits moyens pour détecter le pH est un mélange de Méthylorange et de vert de Bromocrésol dans un rapport 1:5 (en poids).

5. Le gant selon l'une quelconque des revendications 1 à 4, dans lequel un élément d'information à propos du relevé de pH est joint.

6. Le gant selon la revendication 5, dans lequel ledit élément d'information est une échelle de couleur.

7. Le gant selon la revendication 4, dans lequel lesdites valeurs égales ou au-dessus de 4,5 sont définies comme une alerte.

8. Le gant selon l'une quelconque des revendications 1 à 7, dans lequel lesdits moyens pour prélever un échantillon vaginal sont fabriqués dans un matériau approprié pour prélever le fluide vaginal et/ou le mucus.

9. Le gant selon la revendication 8, dans lequel ledit matériau des moyens est une gaze monocouche absorbante non-tissée.

10. Le gant selon l'une quelconque des revendications 1 à 9, qui est fabriqué en vinyle ou en latex.

11. Le gant selon l'une quelconque des revendications 1 à 10, comprenant également un dispositif distinct pour la détermination d'un test supplémentaire utile pour déterminer la présence d'une affection vaginale.

12. Le gant selon la revendication 11, dans lequel ledit test supplémentaire est choisi parmi le groupe constitué d'un test pour les amines volatiles, d'un test pour déterminer la quantité d'acide lactique, d'un test pour la détermination de l'activité d'enzymes d'intérêt comme marqueurs d'un état pathologique et/ou d'autres indicateurs biologiques et d'un test pour déterminer la présence de marqueurs d'infection.

13. Le gant selon l'une quelconque des précédentes revendications 1 à 12, qui est utile pour déterminer la présence d'une affection vaginale choisie parmi le groupe constitué d'une infection vaginale et de vaginite.

14. Le gant selon la revendication 13, dans lequel, ladite infection vaginale est choisie parmi le groupe constitué de la vaginose bactérienne, de la vaginite aérobie, d'infection fongique et d'infection virale.

15. Un kit comprenant : a) au moins un gant selon l'une quelconque des revendications 1 à 14 et des instructions pour son utilisation.

16. Le kit selon la revendication 15, comprenant également :
b) une composition gynécologique ; et optionnellement
c) des instructions pour utiliser le gant a) et la composition b).

17. Le kit selon la revendication 16, dans lequel ladite composition (b) comprend des bactéries lactiques et/ou des bifidobactéries choisies parmi le groupe constitué de *Lactobacillus acidophilus, Lactobacillus buchneri, Lactobacillus casei, Lactobacillus catenaforme, Lactobacillus cellobiosus, Lactobacillus crispatus, Lactobacillus curvatus, Lactobacillus delbrueckii, Lactobacillus jensenii, Lactobacillus leichmannü, Lactobacillus minutus, Lactobacillus paracasei, Lactobacillus plantarum, Lactobacillus rogosae, Lactobacillus salivarius, Lactobacillus brevis, Lactobacillus gasseri, Lactobacillus fermentum, Bifidobacterium adolescentis, Bifidobacterium angulatum, Bifidobacterium bifidum, Bifidobacterium breve, Bifidobacterium catenulatum, Bifidobacterium dentium, Bifidobacterium eriksonii, Bifidobacterium infants, Bifidobacterium longum, Bifidobacterium plantarum, Bifidobacterium pseudo-catenulatum, Bifidobacterium pseudolongum, Streptococcus lactis, Streptococcus raffinolactis, Acidaminococcus fermenta, Cytophaga fermentas, Rhodoferax fermentans, Cellulomonas fermentans, Zymomonas mobilis,* et *Streptococcus thermophilus.*

18. Le kit selon la revendication 17, dans lequel les bactéries lactiques sont choisies dans le groupe constitué des espèces *Lactobacillus brevis* et *Lactobacillus salivarius* subsp. *salicinius* optionnellement en combinaison avec une ou plusieurs espèces de lactobacilles choisies parmi le groupe constitué de *Lactobacillus salivarius* subsp. *salivarius, Lactobacillus jensenii, Laetobaeillus catenaforme, Lactobacillus minutus, Lactobacillus gasseri* et *Laetobaeillus plantarum.*

19. Le kit selon la revendication 18, dans lequel les bactéries lactiques sont une combinaison constituée de *Lactobacillus brevis, Lactobacillus salivarius* subsp. *salicinius* et *Lactobacillus plantarum.*

20. Le kit selon la revendication 17, dans lequel les bactéries lactiques sont une combinaison des espèces *Lactobacillus brevis* et *Lactobacillus salivarius* subsp. *salicinius.*

21. Le kit selon la revendication 17, dans lequel lesdites bactéries lactiques sont une combinaison de (a) un premier composant constitué d'au moins une souche de bactéries lactiques produisant du H₂O₂, et (b) un second composant constitué d'au moins une souche de bactéries lactiques utilisant l'arginine.

22. Le kit selon la revendication 21, dans lequel ladite bactérie lactique est (a) choisie parmi le groupe constitué de *Lactobacillus crispatus, Lactobacillus salivarius* et *Lactobacillus casei,* et le composant (b) est choisi parmi les souches des espèces *Lactobacillus brevis, Lactobacillus gasseri* et *Lactobacillus fermentum.*

23. Le kit selon la revendication 21, dans lequel les bactéries lactiques sont une combinaison des espèces *Lactobacillus brevis* et *Lactobacillus salivarius* subsp. *salicinius.*

24. Le kit selon l'une quelconque des revendications 17 à 23, dans lequel la bactérie lactique est *Lactobacillus brevis* CD2 DSM 11988.

25. Le kit selon l'une quelconque des revendications 21 à 24, dans lequel ladite composition comprend également une ou plusieurs espèces de lactobacilles choisies parmi le groupe constitué de *Lactobacillus salivarius subs. Salivarius, Lactobacillus jensenii, Lactobacillus catenaforme, Lactobacillus minutus,* et *Lactobacillus plantarum.*

26. Le kit selon la revendication 17, dans lequel lesdits lactobacilles consistent en les espèces *Lactobacillus brevis, Lactobacillus casei, Lactobacillus salivarius subs. Salicinius* et *Lactobacillus gasseri.*

27. Le kit selon l'une quelconque des revendications 17 à 26, dans lequel lesdits lactobacilles sont présents à une concentration de 10⁷ à 10¹³ CFU/g.

28. Le kit selon l'une quelconque des revendications 17 à 27, dans lequel la composition comprend également au moins une substance choisie parmi le groupe constitué de vitamines, de bases ammonium quaternaires, de sels minéraux, d'huile d'arbre de thé, de soja et de ses dérivés, d'agents antioxydants, d'agents antiinflammatoires, d'agents antihistaminiques, et de substance libérant du NO.

29. Le kit selon la revendication 17, dans lequel la composition (b) comprend au moins une substance choisie parmi le groupe constitué de l'huile d'arbre de thé et/ou de soja et de ses dérivés.

30. Le kit selon la revendication 29, dans lequel la composition comprend également au moins une substance choisie parmi le groupe constitué de vitamines, de bases ammonium quaternaires, de sels minéraux et d'agents antioxydants.

31. Le kit selon l'une quelconque des revendications 17 à 30, dans lequel ladite composition est sous forme liquide ou d'une crème, d'onguent, de pessaire, de comprimé ou de sachet vaginal.

32. Le kit selon la revendication 31, dans lequel ledit comprimé vaginal comprend au moins une couche de lactobacilles, sous forme liée, pour contrôler la vitesse de libération des bactéries.

33. Le kit selon la revendication 32, dans lequel ledit comprimé comprend au moins deux couches, la couche externe et la couche la plus interne contenant lesdites espèces de lactobacilles, sous forme liée, de façon à ce que la vitesse de libération des bactéries de la couche externe soit supérieure à la vitesse de libération des bactéries de la couche la plus interne.

34. Le kit selon la revendication 33, dans lequel dans ledit comprimé, les bactéries de la couche externe sont libérées dans un laps de temps d'environ 10 à 25 minutes, alors que les bactéries de la couche la plus interne sont libérées ultérieurement dans un laps de temps d'environ 25 à 50 minutes.

35. Le kit selon l'une quelconque des revendications 16 à 34, comprenant également (d) une composition pour le traitement des vaginoses bactériennes ou des vaginites aérobies et pour la recolonisation de l'environnement vaginal.

36. Le kit selon la revendication 35, dans lequel ladite composition (d) comprend une combinaison d'un agent antibactérien et d'un agent antifongique.

37. Le kit selon la revendication 36, dans lequel ledit agent antibactérien est le métronidazole et ledit agent antifongique est le fucanozole.

38. Le kit selon l'une quelconque des revendications 35 à 37, comprenant également des instructions pour l'utilisation du gant (a), de la composition (b) et de la composition (d).

39. Une méthode pour la détermination de la présence d'une affection vaginale, comprenant les étapes de :
(i) mise en contact des moyens de prélèvement du gant selon l'une quelconque des revendications 1 à 14 qui a été utilisé pour obtenir l'échantillon vaginal avec les moyens de détection du Ph,
(ii) lecture de la substance signal indicatrice exposée et détermination de la présence supposée de ladite affection vaginale.

40. La méthode selon la revendication 39, dans laquelle ladite affection est choisie parmi le groupe constitué des infections vaginales, de la vaginite et de la dyspareunie.

41. La méthode selon la revendication 40, dans laquelle ladite infection est choisie parmi le groupe constitué de la vaginose bactérienne, de la vaginite aérobie, d'infection fongique et d'infection virale.

42. Utilisation d'un gant selon l'une quelconque des revendications 1 à 14 pour la détection d'une affection vaginale.

43. Utilisation d'un kit selon l'une quelconque des revendications 15 à 38 pour la détection d'une affection vaginale.

44. L'utilisation selon la revendication 43, dans laquelle ladite affection est choisie parmi le groupe constitué des infections vaginales, de la vaginite et de la dyspareunie.

45. L'utilisation selon la revendication 44, dans laquelle ladite infection est choisie parmi le groupe constitué de la vaginose bactérienne, de la vaginite aérobie, d'infection fongique et d'infection virale.
